# EUROPEAN PATENT APPLICATION

(11) **EP 3 296 841 A1**
(43) Date of publication of application: **21.03.2018**
(21) Application number: 15891768.2
(22) Date of filing: 08.05.2015
(51) Int. Cl.: G06F 3/01, H04M 1/00

(54) **ELECTRONIC APPARATUS, DETECTION METHOD, AND DETECTION PROGRAM**

(71) Applicant: Fujitsu Limited, Kawasaki-shi, Kanagawa 211-8588 (JP)
(72) Inventor: SUGIMOTO, Takanao, Kawasaki-shi Kanagawa 211-8588 (JP); NEGORO, Hiroshi, Kawasaki-shi Kanagawa 211-8588 (JP)
(74) Representative: Haseltine Lake LLP
(86) International application number: PCT/JP2015/063372
(87) International publication number: WO 2016/181442

(57) **Abstract**

A wearable terminal (10) calculates an acceleration amount in the vertical direction by using an acceleration value. The wearable terminal (10) calculates a change amount in an atmospheric pressure value by using atmospheric pressure values. Further, the wearable terminal (10) multiplies the acceleration amount in the vertical direction by the change amount in the atmospheric pressure value. After that, the wearable terminal (10) detects a change amount in the vertical direction on the basis of the calculated multiplication result. For example, when the multiplication result is a positive value, the wearable terminal (10) sets the change amount in the vertical direction as "0". When the multiplication result is a negative value, the wearable terminal (10) sets the multiplication result as the change amount in the vertical direction.

## Description

### [Technical Field]

The present invention relates to an electronic device, a detecting method, and a detecting computer program.

### [Background Art]

Conventionally, various kinds of electronic devices such as portable terminals and wearable terminals have various types of small sensors installed therein. Applications that have become popular include those that, by using values obtained by such sensors, execute a life-log function to review changes in our daily life and a health support function. For example, an example of such electronic devices is configured to measure a change amount in the horizontal direction or a change amount in the vertical direction of the electronic device, by using an acceleration value.

In recent years, a technique has been known by which a change amount in the vertical direction is detected with a high level of precision by using an atmospheric pressure sensor. For example, an example of such electronic devices is configured to measure an atmospheric pressure value by using an atmospheric pressure sensor installed therein and to measure a change amount in the vertical direction on the basis of a change in the atmospheric pressure value.

### [Citation List]

### [Patent Literature]

Patent Document 1: Japanese Laid-open Patent Publication No. 2013-92923

### [Summary of invention]

### [Technical Problem]

However, when the technique described above is used, an erroneous detection may occur when detecting the change amount in the vertical direction. For example, when a drastic change has occurred in the atmospheric pressure in a room due to opening/closing of a door, a change amount indicating that the electronic device has moved in a vertical direction may erroneously be detected, although the electronic device has not moved in a vertical direction.

It is an object of at least one aspect of the present disclosure to provide an electronic device, a detecting method, and a detecting computer program that are able to prevent erroneous detections of change amounts in the vertical direction.

### [Solution to Problem]

According to an aspect of an embodiment, an electronic device includes a first calculating unit that calculates an acceleration amount in a vertical direction by using an acceleration value; a second calculating unit that calculates a change amount in an atmospheric pressure value by using atmospheric pressure values; a multiplying unit that multiplies the acceleration amount in the vertical direction by the change amount in the atmospheric pressure value; and a detecting unit that detects a change amount in the vertical direction on a basis of a multiplication result calculated by the multiplying unit.

### [Advantageous Effects of Invention]

According to at least one aspect of the embodiments, it is possible to prevent erroneous detections of change amounts in the vertical direction.

### [Brief Description of Drawings]

FIG. 1 is a diagram illustrating an example of an overall configuration of a system according to a first embodiment.
FIG. 2 is a diagram illustrating an example of a hardware configuration of a wearable terminal.
FIG. 3 is a functional block diagram illustrating a functional configuration of the wearable terminal.
FIG. 4 is a drawing illustrating an example of information stored in a threshold value database (DB).
FIG. 5 is a drawing for explaining an example of a posture specifying process.
FIG. 6 is a flowchart illustrating a flow in a change amount calculating process.
FIG. 7 is a flowchart illustrating a flow in the posture specifying process.
FIG. 8 is a drawing for explaining an example in which erroneous detections caused by an external disturbance are prevented.
FIG. 9 is a drawing for explaining an example in which a change amount in the height is corrected.
FIG. 10 is a drawing for explaining an example in which it is detected that the user is moving on stairs.

### [Description of Embodiments]

Exemplary embodiments of an electronic device, a detecting method, and a detecting computer program disclosed herein will be explained in detail below, with reference to the accompanying drawings. The present disclosure is not limited by the embodiments described below. Further, it is possible to combine any of the embodiments together as appropriate, as long as no conflict occurs.

### First Embodiment

### [Overall configuration]

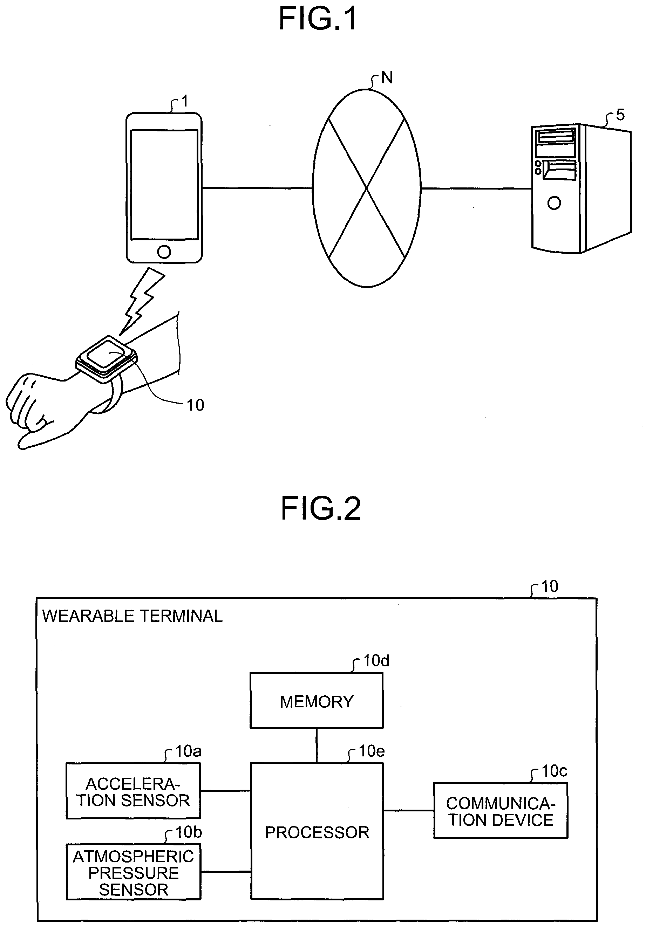

FIG. 1 is a diagram illustrating an example of an overall configuration of a system according to a first embodiment. As illustrated in FIG. 1, in the system, a portable terminal 1 and a cloud server 5 are connected to each other via a network N, while the portable terminal 1 and a wearable terminal 10 are connected to each other by near field wireless communication.

The network N may be wired or wireless. It is possible to configure the network N by using an arbitrary type of communication network such as the Internet, a Local Area Network (LAN), a Virtual Private Network (VPN), or the like. Further, the near field wireless communication may be of a contact-requiring type or a contactless type. It is possible to configure the near field wireless communication by using Bluetooth (a registered trademark) or a Near Field Communication (NFC) scheme.

The portable terminal 1 is an example of a mobile terminal possessed by a user and may be, for example, a smartphone, a laptop personal computer, or the like. The portable terminal 1 is configured to receive posture information of the user or the like from the wearable terminal 10 and to transmit the received posture information to the cloud server 5. Further, the portable terminal 1 is configured to receive various types of information from the cloud server 5 and to cause the received information to be displayed on a display device or the like.

The cloud server 5 is configured to detect an abnormality such as the user's falling, on the basis of the posture information received from the portable terminal 1. For example, when having detected that the user being monitored has fallen, the cloud server 5 transmits an electronic mail or the like indicating that the monitored user has fallen, to a contact address that is designated in advance. Further, the cloud server 5 is also capable of transmitting a web screen or the like displaying that the monitored user has fallen.

The wearable terminal 10 is an example of an electronic device attached to (or worn by) the user possessing the portable terminal 1. The wearable terminal 10 includes various types of sensors such as an acceleration sensor, a gyro sensor, a geomagnetic sensor, an atmospheric pressure sensor, and/or the like. The wearable terminal 10 is configured to calculate an acceleration amount in the vertical direction by using an acceleration value and to calculate a change amount in the atmospheric pressure value by using atmospheric pressure values. Further, the wearable terminal 10 is further configured to multiply the acceleration amount in the vertical direction by the change amount in the atmospheric pressure value and to detect a change amount in the vertical direction on the basis of the result of the multiplication.

For example, when the multiplication result obtained by multiplying the acceleration amount in the vertical direction by the change amount in the atmospheric pressure value is a positive value, the wearable terminal 10 detects the change amount in the vertical direction as "0". When the multiplication result is a negative value, the wearable terminal 10 detects the negative value as the change amount in the vertical direction. As a result, the wearable terminal 10 is able to prevent erroneous detections in the change amount in the vertical direction.

### A hardware configuration

FIG. 2 is a diagram illustrating an example of a hardware configuration of the wearable terminal. The portable terminal 1 has a hardware configuration similar to that of a commonly-used portable terminal. The cloud server 5 has a hardware configuration similar to that of a commonly-used server.

As illustrated in FIG. 2, the wearable terminal 10 includes an acceleration sensor 10a, an atmospheric pressure sensor 10b, a communication device 10c, a memory 10d, and a processor 10e. The pieces of hardware described here are merely example. The wearable terminal 10 may further include one or more other pieces of hardware such as a display device.

The acceleration sensor 10a is a sensor configured to detect an acceleration value (m/s²) and may be configured with a three-axis sensor, for example. The acceleration sensor 10a is configured, for example, to measure an acceleration value (an acceleration vector) on each of the x-, y-, and z- axes and to output measured results to the processor 10e. The atmospheric pressure sensor 10b is a sensor configured to measure an atmospheric pressure value (hpa) and is configured to output a measured result to the processor 10e.

The communication device 10c is a device configured to perform near field wireless communication. For example, the communication device 10c is configured to connect to the portable terminal 1 by using Bluetooth (a registered trademark) and to transmit/receive various types of information. The memory 10d is an example of a storage device configured to store therein various types of databases and computer programs.

The processor 10e is a processing unit configured to control the entirety of the wearable terminal 10 and may be configured with a Central Processing Unit (CPU), for example. The processor 10e is configured to execute a computer program used for performing various processes (explained later) and to execute various types of processes that perform the same processes as those of the processing unit (explained later).

### [Functional configuration]

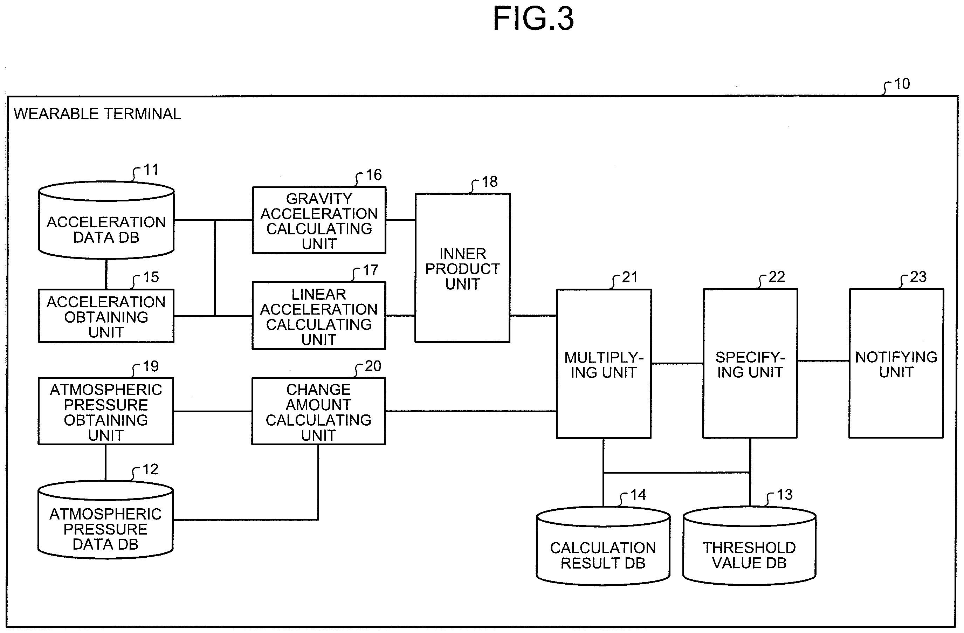

FIG. 3 is a functional block diagram illustrating a functional configuration of the wearable terminal. As illustrated in FIG. 3, the wearable terminal 10 includes an acceleration data database (DB) 11, an atmospheric pressure data DB 12, a threshold value DB 13, a calculation result DB 14, an acceleration obtaining unit 15, a gravity acceleration calculating unit 16, a linear acceleration calculating unit 17, and an inner product unit 18. Further, the wearable terminal 10 also includes an atmospheric pressure obtaining unit 19, a change amount calculating unit 20, a multiplying unit 21, an specifying unit 22, and a notifying unit 23.

In this situation, the acceleration data DB 11, the atmospheric pressure data DB 12, the threshold value DB 13, and the calculation result DB 14 are stored in a storage device such as the memory 10d or a hard disk. The acceleration obtaining unit 15, the gravity acceleration calculating unit 16, the linear acceleration calculating unit 17, the inner product unit 18, the atmospheric pressure obtaining unit 19, the change amount calculating unit 20, the multiplying unit 21, the specifying unit 22, and the notifying unit 23 are examples of electronic circuits included in the processor 10e and/or examples of the processes executed by the processor 10e.

The acceleration data DB 11 is a database configured to store therein the three-axis acceleration data (which hereinafter may be referred to as "acceleration values" or "acceleration vectors") measured by the acceleration sensor 10a. For example, the acceleration data DB 11 stores therein pieces of measured three-axis acceleration data in a time series.

The atmospheric pressure data DB 12 is a database configured to store therein atmospheric pressure values measured by the atmospheric pressure sensor 10b. For example, the atmospheric pressure data DB 12 stores therein the measured atmospheric pressure values in a time series.

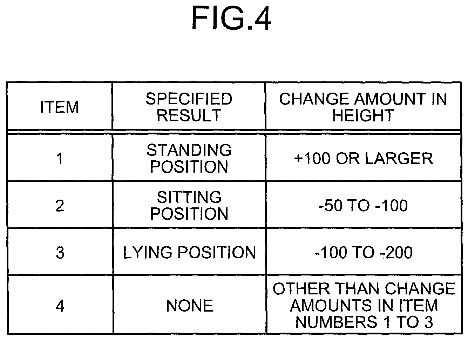

The threshold value DB 13 is a database configured to store therein threshold values for change amounts in the height used for specifying the posture of the user, i.e., threshold values for change amounts in the vertical direction. FIG. 4 is a drawing illustrating an example of the information stored in the threshold value DB. As illustrated in FIG. 4, the threshold value DB 13 stores therein "items, specified results, and change amounts in the height" so as to be kept in correspondence with one another.

In the present example, stored under the "item" are item numbers identifying records. Stored under the " specified result" are pieces of information indicating the postures of the relevant user. Stored under the "change amount in the height" are threshold values used for specifying the posture of the user. In an example illustrated in FIG. 4, item number 1 indicates that when the change amount in the height is "+100" or larger, the posture of the user is specified to be a "standing position". Item number 2 indicates that when the change amount in the height is in the range of "-50 to -100", the posture of the user is specified to be a "sitting position".

The calculation result DB 14 is a database configured to store therein multiplication results calculated by the multiplying unit 21 (explained later). More specifically, the calculation result DB 14 stores therein the multiplication results that are calculated by the multiplying unit 21 and that are represented by scalar values used by the specifying unit 22 to perform the posture specifying process. Further, the calculation result DB 14 is also able to store therein, in a time series, pieces of posture information resulting from the specifying process.

The acceleration obtaining unit 15 is a processing unit configured to obtain an acceleration value. More specifically, the acceleration obtaining unit 15 occasionally obtains the acceleration values on the x-, y-, and z- axes measured by the acceleration sensor 10a from the acceleration sensor 10a and stores the obtained acceleration values into the acceleration data DB 11.

The gravity acceleration calculating unit 16 is a processing unit configured to calculate a gravity acceleration value. For example, the gravity acceleration calculating unit 16 reads the acceleration vector from the acceleration data DB 11 and calculates only a value (a bias amount) in the gravity direction of the acceleration, by using a low-pass filter, a moving average process, or the like. Further, the gravity acceleration calculating unit 16 outputs the calculation result to the inner product unit 18, as the gravity acceleration value.

The linear acceleration calculating unit 17 is a processing unit configured to calculate a linear acceleration value. For example, the linear acceleration calculating unit 17 reads the acceleration vector from the acceleration data DB 11 and calculates values of the acceleration in the directions other than the gravity direction, by using a high-pass filter, a linear acceleration method, or the like. After that, the linear acceleration calculating unit 17 outputs the calculation result to the inner product unit 18, as the linear acceleration value.

The inner product unit 18 is a processing unit configured to calculate an inner product of the gravity acceleration value and the linear acceleration value. For example, the inner product unit 18 calculates an acceleration amount in the vertical direction by calculating an inner product of the gravity acceleration value received from the gravity acceleration calculating unit 16 and the linear acceleration value received from the linear acceleration calculating unit 17. Further, the inner product unit 18 outputs the calculated acceleration amount in the vertical direction to the multiplying unit 21.

The atmospheric pressure obtaining unit 19 is a processing unit configured to obtain an atmospheric pressure value. More specifically, the atmospheric pressure obtaining unit 19 occasionally obtains atmospheric pressure values measured by the atmospheric pressure sensor 10b from the atmospheric pressure sensor 10b and stores the obtained atmospheric pressure values into the atmospheric pressure data DB 12.

The change amount calculating unit 20 is a processing unit configured to calculate a change amount in the atmospheric pressure value. More specifically, the change amount calculating unit 20 reads the atmospheric pressure value from the atmospheric pressure data DB 12, calculates a change amount in the atmospheric pressure value by using a low-pass filter, a moving average process, or the like, and outputs the calculation result to the multiplying unit 21. For example, the change amount calculating unit 20 obtains atmospheric pressure values from the current time to 4 second ago, calculates an average value thereof, and further calculates a change amount in the average value. Further, the change amount calculating unit 20 is also capable of calculating a change amount in the atmospheric pressure value on the basis of the difference between the atmospheric pressure value observed 4 seconds ago and the atmospheric pressure value at the current point in time (the most up-to-date atmospheric pressure value).

The multiplying unit 21 is a processing unit configured to multiply the acceleration amount in the vertical direction by the change amount in the atmospheric pressure value. More specifically, the multiplying unit 21 multiplies the acceleration amount in the vertical direction received from the inner product unit 18 by the change amount in the atmospheric pressure value received from the change amount calculating unit 20. In this situation, by using a delay circuit or the like provided therewith, the multiplying unit 21 performs the calculation by selecting an acceleration amount in the vertical direction and a change amount in the atmospheric pressure value that are calculated on the basis of pieces of information observed at mutually the same point in time.

For example, when the result obtained by multiplying the acceleration amount in the vertical direction by the change amount in the atmospheric pressure value is a positive value, the multiplying unit 21 calculates a change amount in the height as "0" and further stores the multiplication result into the calculation result DB 14. On the contrary, when the result obtained by multiplying the acceleration amount in the vertical direction by the change amount in the atmospheric pressure value is a negative value, the multiplying unit 21 stores the multiplication result into the calculation result DB 14, as a change amount in the height.

In other words, when the posture of the user changes from a standing position to either a sitting position or lying position, the acceleration amount in the vertical direction occurs in a downward direction, while the change amount in the atmospheric pressure value increases at that time. Accordingly, the multiplication result at that time is a negative value. After that, the acceleration amount occurs in an upward direction, while the change amount in the atmospheric pressure value at that time remains increased. Accordingly, the multiplication result at that time is a positive value. In other words, it is possible to calculate the change amount only during the time period when the positions of the sensors are changing vertically downward while the acceleration is occurring in the downward direction. With this arrangement, even when the atmospheric pressure in a room rapidly changes due to opening/closing of a door, for example, the change amount in the height is "0", as long as the acceleration has not changed. Accordingly, because the acceleration does not change even a change in the atmospheric pressure value is detected due to an impact of the wind, the change amount in the height is determined as "0".

In contrast, when the change amount in the atmospheric pressure value is a negative value, while the acceleration amount in the vertical direction exhibits a positive change amount, the multiplying unit 21 calculates the absolute value of the calculation result and stores the absolute value into the calculation result DB 14 as a change amount in the height. In other words, when the posture of the user changes from either a sitting position or a lying position to a standing position, it is possible to calculate the change amount only during the time period when the positions of the sensors are changing vertically upward while the acceleration is occurring in the upward direction.

The specifying unit 22 is a processing unit configured to specify the posture of the user according to the multiplication result obtained by the multiplying unit 21. More specifically, the specifying unit 22 reads the change amount in the height stored in the calculation result DB 14 and specifies the posture of the user by comparing the read change amount in the height with the threshold values stored in the threshold value DB 13. After that, the specifying unit 22 informs the notifying unit 23 of the specified posture of the user.

For example, when the change amount in the height is +100 or larger, the specifying unit 22 specifies the posture of the user as a standing position. Further, when the change amount in the height is in the range from -50 to -100, the specifying unit 22 specifies the posture of the user as a sitting position. Further, when the change amount in the height is in the range from -100 to -200, the specifying unit 22 specifies the posture of the user as a lying position. When a detected change amount in the height is different from any of the above, the specifying unit 22 determines that it is impossible to specify the posture of the user.

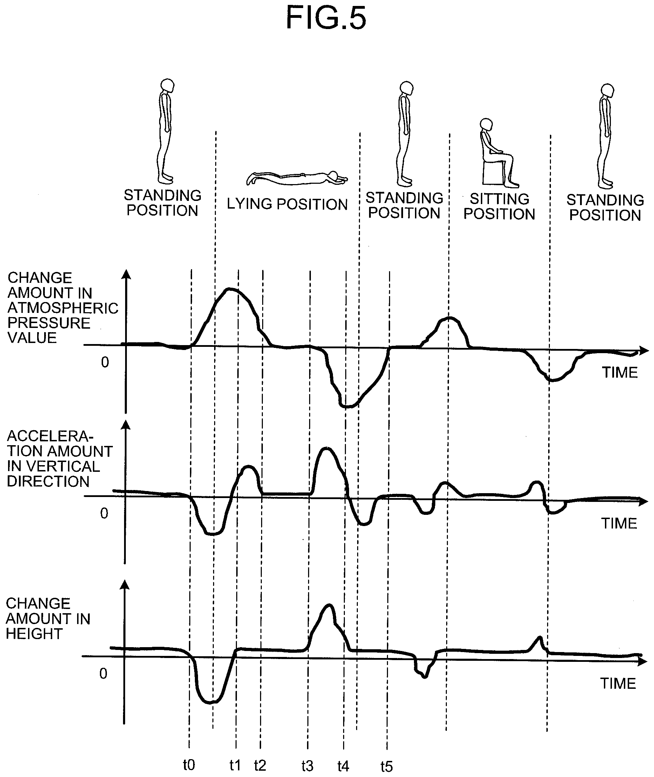

Next, specific examples will be explained. FIG. 5 is a drawing for explaining an example of the posture specifying process. In FIG. 5, the vertical axis of the chart expresses change amounts in the atmospheric pressure value, acceleration amounts in the vertical direction, and change amounts in the height, whereas the horizontal axis expresses time. In the present example, a situation starting with a standing position will be explained.

As illustrated in FIG. 5, during the time period from the time t0 to the time t1, because the change amount in the atmospheric pressure value is a positive value while the acceleration amount in the vertical direction is a negative value, the multiplication result obtained by the multiplying unit 21 is a negative value. In this situation, the specifying unit 22 determines that the change amount in the height represented by the multiplication result is in the range from -100 to -200 and specifies that the posture of the user has shifted into a lying position.

Subsequently, during the time period from the time t1 to the time t2, because the change amount in the atmospheric pressure value is a positive value, while the acceleration amount in the vertical direction is a positive value, the multiplication result obtained by the multiplying unit 21 is a positive value, and the change amount in the height is therefore "0". In this situation, because the change amount in the height is "0", the specifying unit 22 specifies that no change has occurred in the posture of the user and that the user is in the lying position.

After that, during the time period from the time t2 to the time t3, because the change amount in the atmospheric pressure value is approximately "0", while the acceleration amount in the vertical direction is approximately "0", the multiplication result obtained by the multiplying unit 21 is "0", and the change amount in the height is therefore approximately "0". In this situation, because the change amount in the height is "0", the specifying unit 22 specifies that no change has occurred in the posture of the user and that the user is in the lying position.

Further, during the time period from the time t3 to the time t4, because the change amount in the atmospheric pressure value is a negative value, while the acceleration amount in the vertical direction is a positive value, the multiplying unit 21 calculates the absolute value of the multiplication result as a change amount in the height. In this situation, the specifying unit 22 determines that the change amount in the height is +100 or larger and specifies that the posture of the user has shifted from the lying position to a standing position.

Subsequently, during the time period from the time t4 to the time t5, because the change amount in the atmospheric pressure value is a negative value, while the acceleration amount in the vertical direction is a negative value, the multiplication result obtained by the multiplying unit 21 is a positive value, and the change amount in the height is therefore "0". In this situation, because the change amount in the height is "0", the specifying unit 22 specifies that no change has occurred in the posture of the user and that the user is in the standing position.

In this manner, the specifying unit 22 occasionally specifies the posture of the user by comparing the change amounts in the height calculated by the multiplying unit 21 with the threshold values. As a result, the specifying unit 22 is able to occasionally detect the changes in the posture of the user.

Returning to the description of FIG. 3, the notifying unit 23 is a processing unit configured to notify a superordinate application that requested the execution of the user posture determination of the specified result. Alternatively, the notifying unit 23 may be configured, when the posture of the user has changed, to notify the superordinate application that the posture has changed. When having received posture information related to the posture of the user, the superordinate application transmits the posture information to the portable terminal 1, by using Bluetooth or the like.

### [Procedure of Processing]

Next, various types of processes performed by the wearable terminal 10 will be explained. In the present example, a change amount calculating process and a posture specifying process will be explained.

### A change amount calculating process

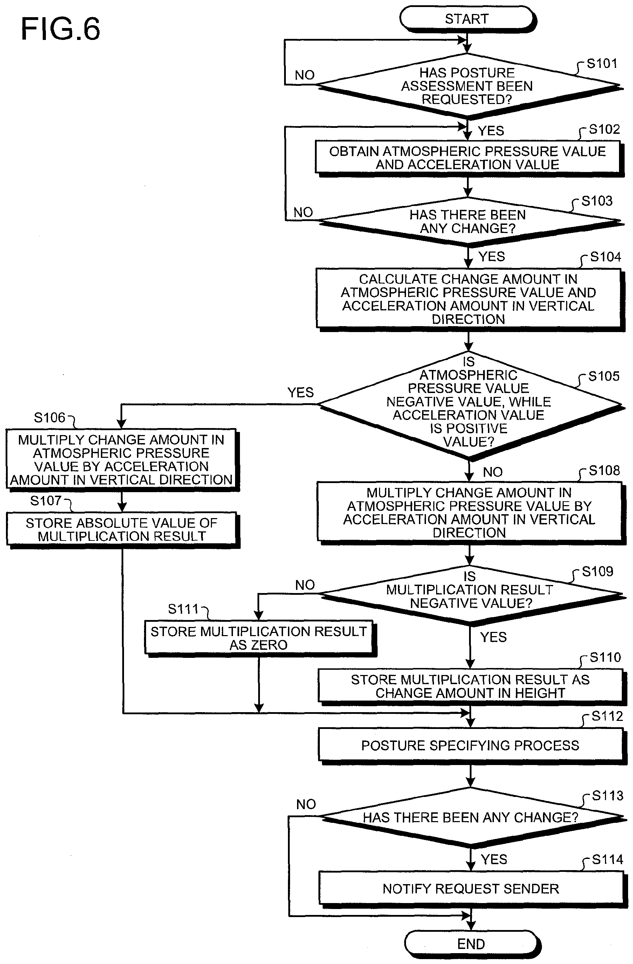

FIG. 6 is a flowchart illustrating a flow in the change amount calculating process. As illustrated in FIG. 6, when having received a posture specified request from a superordinate application selected by the user (step S101: Yes), the acceleration obtaining unit 15 obtains an acceleration value, whereas the atmospheric pressure obtaining unit 19 obtains an atmospheric pressure value (step S102).

In this situation, when there has not been any change from either the most recently obtained atmospheric pressure value or the most recently obtained acceleration value (step S103: No), the processes at step S102 and thereafter will be repeatedly performed. On the contrary, when there has been a change from either the most recently obtained atmospheric pressure value or the most recently obtained acceleration value (step S103: Yes), an acceleration amount in the vertical direction and a change amount in the atmospheric pressure value are calculated (step S104).

After that, when the change amount in the atmospheric pressure value is a negative value, while the acceleration amount in the vertical direction is a positive value (step S105: Yes), the multiplying unit 21 multiplies the change amount in the atmospheric pressure value by the acceleration amount in the vertical direction (step S106) and stores the absolute value of the multiplication result into the calculation result DB 14 as a change amount in the height (step S107). Subsequently, the process at step S112 explained later is performed.

On the contrary, when the condition is not satisfied where the change amount in the atmospheric pressure value is a negative value, while the acceleration amount in the vertical direction is a positive value (step S105: No), the multiplying unit 21 multiplies the change amount in the atmospheric pressure value by the acceleration amount in the vertical direction (step S108).

Further, when the multiplication result is a negative value (step S109: Yes), the multiplying unit 21 stores the multiplication result into the calculation result DB 14 as a change amount in the height (step S110). On the contrary, when the multiplication result is a positive value (step S109: No), the multiplying unit 21 stores a change amount in the height into the calculation result DB 14 as "0" (step S111).

After that, the specifying unit 22 performs an specifying process (step S112). When the posture of the user has changed (step S113: Yes), the notifying unit 23 notifies the superordinate application which made the request that the posture of the user has changed (step S114). When the posture of the user has not changed (step S113: No), the process is ended.

### A posture specifying process

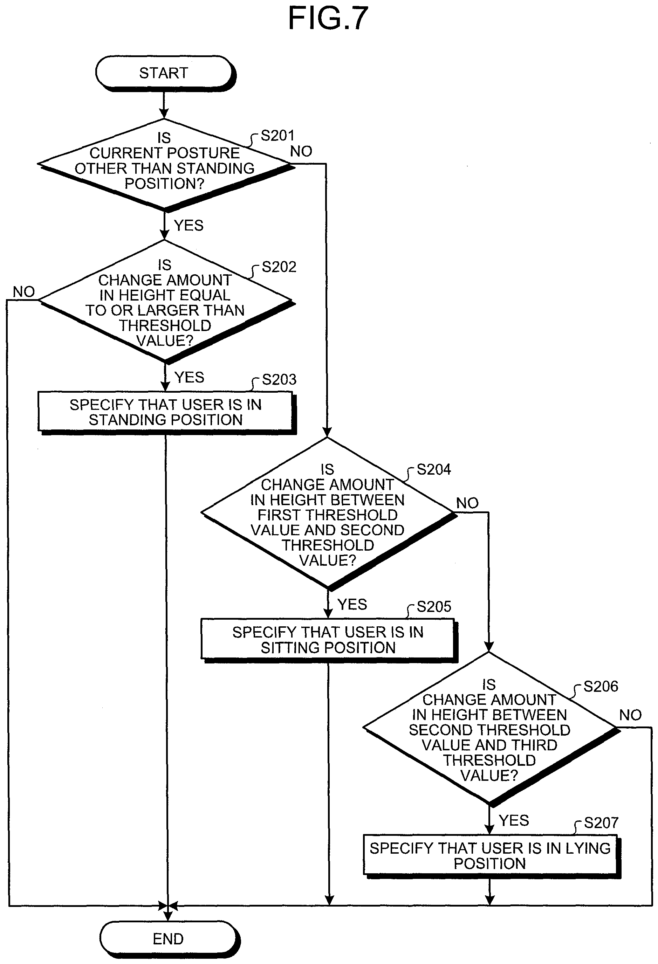

FIG. 7 is a flowchart illustrating a flow in the posture specifying process. As illustrated in FIG. 7, when the current posture is a position other than the standing position (step S201: Yes), while the change amount in the height is equal to or larger than the threshold value (step S202: Yes), the specifying unit 22 specifies that the posture of the user is a standing position (step S203). In contrast, when the current posture is a position other than the standing position, but the change amount in the height is smaller than the threshold value (step S202: No), the specifying unit 22 determines that it is impossible to make an determination and ends the process.

In contrast, when the current posture is a standing position (step S201: No), while the change amount in the height is in the range from the first threshold value to the second threshold value (step S204: Yes), the specifying unit 22 specifies that the posture of the user is a sitting position (step S205).

In contrast, when the current posture is a standing position, while the change amount in the height is not in the range from the first threshold value to the second threshold value (step S204: No), and the change amount in the height is in the range between the second threshold value and the third threshold value (step S206: Yes), the specifying unit 22 specifies that the posture of the user is a lying position (step S207). In contrast, when the change amount in the height is equal to or smaller than the third threshold value (step S202: No), the specifying unit 22 determines that it is impossible to make an determination and ends the process.

### [Advantageous effects]

As explained above, the wearable terminal 10 is able to calculate the change amount in the atmospheric pressure observed while the acceleration is occurring in the vertical direction due to the change in the posture of the user. When the posture of the user has not changed in the vertical direction so that no acceleration has occurred in the vertical direction, the wearable terminal 10 does not perform the posture change specifying process. With this arrangement, the wearable terminal 10 is able to prevent erroneous detections of changes in the posture caused by changes in the atmospheric pressure value due to changes in the atmospheric pressure in the room or impacts of wind or the like. Accordingly, the wearable terminal 10 is able to improve the level of precision of the determinations. Further, regardless of the orientation in which the wearable terminal 10 is worn, the wearable terminal 10 is able to uniformly calculate the acceleration amount in the vertical direction with respect to the gravity direction. Accordingly, the wearable terminal 10 is able to specify the posture without being dependent on the orientation in which the wearable terminal 10 is worn.

Further, when the posture changes from either a sitting position or a lying position to a standing position, the wearable terminal 10 is able to calculate the change amount only during the time period when the atmospheric pressure value is changing upward in the vertical direction while the acceleration is occurring upward. Further, the wearable terminal 10 is able to distinguish between when the user changes from a standing position to a sitting position and when the user changes from a standing position to a lying position, on the basis of the change amounts in the height.

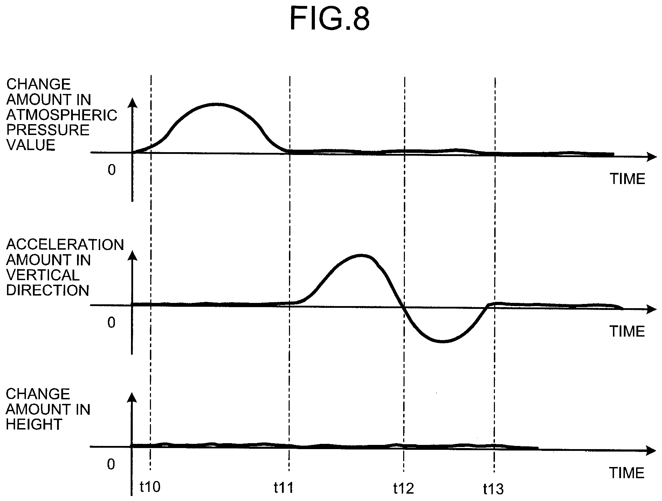

Next, an example in which erroneous detections caused by an external disturbance are prevented will be explained. FIG. 8 is a drawing for explaining an example in which erroneous detections caused by an external disturbance are prevented. Because the explanation of the chart in FIG. 8 is the same as that in FIG. 5, the explanation will be omitted.

The time period from the time t10 to the time t11 in FIG. 8 corresponds to a situation where only the atmospheric pressure instantaneously changed due to opening/closing of a window, for example. In that situation, because the change amount in the atmospheric pressure value is a positive value, while the acceleration amount in the vertical direction is either approximately "0" or a positive value, the wearable terminal 10 calculates a change amount in the height as "0". In this manner, when the atmospheric pressure alone has changed, the wearable terminal 10 is able to detect that the posture of the user has not changed.

Further, the time period from the time t11 to the time t12 in FIG. 8 corresponds to a situation where, for example, the wrist on which the wearable terminal 10 is worn has instantaneously been moved. In that situation, because the change amount in the atmospheric pressure value is approximately "0", while the acceleration amount in the vertical direction is a positive value, the wearable terminal 10 calculates the change amount in the height as "0". Similarly, the time period from the time t12 to the time t13 in FIG. 8 corresponds to the situation where, for example, the wrist on which the wearable terminal 10 is worn has instantaneously been moved. In that situation, because the change amount in the atmospheric pressure value is "0", while the acceleration amount in the vertical direction is a negative value, the wearable terminal 10 calculates the change amount in the height as "0". In this manner, when the acceleration amount in the vertical direction alone has changed, the wearable terminal 10 is able to detect that no change has occurred in the posture of the user.

As explained above, the wearable terminal 10 is able to specify the posture on the basis of the magnitude of the change amount in the height and is able to specify the posture with a high level of precision without being dependent on the orientation in which the wearable terminal 10 is worn and without being affected by changes in the pressure in the room or impacts of wind.

### Second Embodiment

For example, due to erroneous detections of various types of sensors and the like, the wearable terminal 10 may obtain, in some situations, sensor values that have no physical reasons. Even in those situations, the wearable terminal 10 is able to properly correct the change amount in the height and to prevent erroneous detections of the posture of the user. Next, a process of correcting a change amount in the height will be explained.

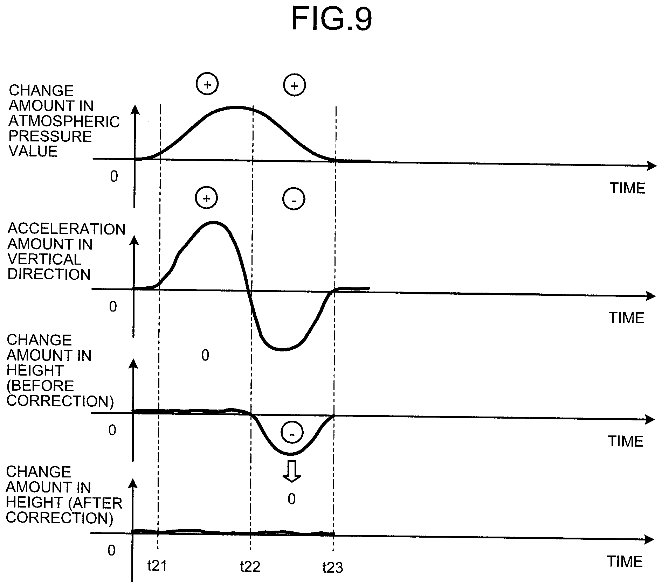

FIG. 9 is a drawing for explaining an example in which a change amount in the height is corrected. Because the explanation of the chart in FIG. 9 is the same as that in FIG. 5, the explanation will be omitted. As illustrated in FIG. 9, during the time period from the time t21 to the time t22, because the wearable terminal 10 detects a change amount in the atmospheric pressure value as a positive value and an acceleration amount in the vertical direction as a positive value, the wearable terminal 10 calculates the change amount in the height as "0".

Subsequently, during the time period from the time t22 to the time t23, the wearable terminal 10 detects the change amount in the atmospheric pressure value as a positive value and the acceleration amount in the vertical direction as a negative value. The wearable terminal 10 thus calculates the multiplication result as a change amount in the height. However, physically speaking, there is a low possibility that such a phenomenon may occur where the acceleration amount in the vertical direction is a negative value, although the change amount in the atmospheric pressure value is a positive value. In other words, generally speaking, when the user changes from a standing position to either a sitting position or a lying position or when the user changes from a sitting position to a lying position, the acceleration amount in the vertical direction changes into a negative value and subsequently changes into a positive value. Further, generally speaking, when the user changes from either a sitting position or a lying position to a standing position, the acceleration amount in the vertical direction changes into a positive value and subsequently changes into a negative value, but the change amount in the atmospheric pressure value is a negative value. Accordingly, during the time period from the time t22 to the time t23, the change amounts detected by the various sensors are unwanted sensor values, which would lead to an erroneous detection of the posture of the user without an adjustment.

To cope with this situation, when the change amount in the atmospheric pressure value and the acceleration amount in the vertical direction are both "0", and subsequently, a positive change amount in the atmospheric pressure value and a positive acceleration amount in the vertical direction are both detected, before a positive change amount in the atmospheric pressure value and a negative acceleration value are detected, the wearable terminal 10 calculates the change amount in the height in these time periods as "0". In the example illustrated in FIG. 9, the wearable terminal 10 calculates the change amount in the height during the time period from the time t22 to the time t23 as "0". With this arrangement, the wearable terminal 10 is able to prevent erroneous detections of the posture of the user, even when unwanted sensor values are detected.

### Third Embodiment

In the embodiments described above, the example is explained in which the wearable terminal 10 specifies the posture of the user as one selected from among: the standing position, the sitting position, and the lying position; however, possible embodiments are not limited to this example. For instance, the wearable terminal 10 is also capable to detecting that the user is moving on stairs. Thus, a process of detecting stairs will be explained as a third embodiment.

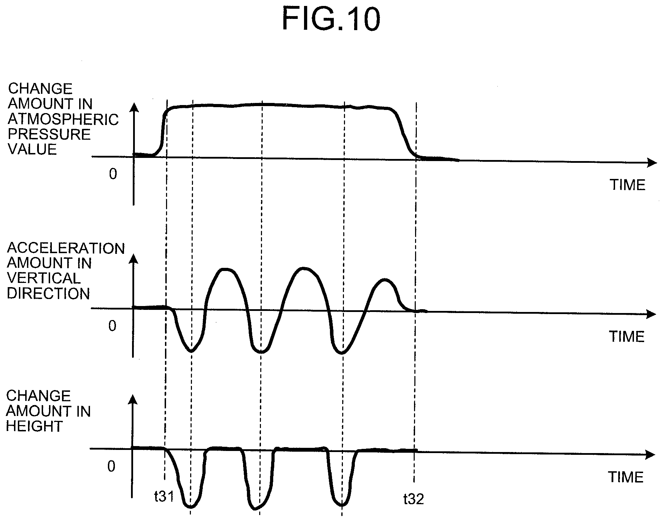

FIG. 10 is a drawing for explaining an example in which it is detected that the user is moving on stairs. Because the explanation of the chart in FIG. 10 is the same as that in FIG. 5, the explanation will be omitted. As illustrated in FIG. 10, during the time period from the time t31 to the time t32, the wearable terminal 10 detects change amounts in the atmospheric pressure value that are constant. Further, during that period, the wearable terminal 10 detects acceleration amounts in the vertical direction that alternate between a negative value and a positive value. In that situation, during the time period from the time t31 to the time t32, the wearable terminal 10 regularly detects change amounts in the height.

Accordingly, when a change amount in the height that is in the range from -100 to -200 is detected three or more times, for example, during a time period in which constant change amounts are detected in the atmospheric pressure value, the wearable terminal 10 specifies that the user is either moving on stairs or climbing up or down a ladder.

Further, when a change amount in the height that is equal to or smaller than -500 is detected three or more times, for example, during a time period in which constant change amounts are detected in the atmospheric pressure value, the wearable terminal 10 specifies that the user is taking an elevator.

As explained above, the wearable terminal 10 is able to detect that the user is moving on stairs or taking an elevator, by using the change amounts in the height during the time period in which the constant change amounts are detected in the atmospheric pressure value.

### Fourth Embodiment

Some exemplary embodiments of the present disclosure have thus been explained; however, it is possible to carry out the present disclosure in various different modes besides those explained above.

### [The prescribed value]

In the embodiments described above, the example is explained in which the wearable terminal 10 determines the change amount in the height to be "0", when the multiplication result obtained by multiplying the change amount in the atmospheric pressure value by the acceleration amount in the vertical direction is a positive value; however, possible embodiments are not limited to this example. For instance, "0" is merely an example, and the change amount in the height may be set to a value designated in advance. Further, although the wording "approximately 0" is used in the embodiments above, the range considered as "0" may arbitrarily be set. For example, for acceleration values, the range 0±5 (m/s²) may be considered as 0. For atmospheric pressure values, the range 0±20 (hpa) may be considered as 0.

### [Distribution and integration of functions]

In the embodiments above, the example is explained in which the wearable terminal 10 performs the sensor value measuring process, the height change amount calculating process, and the posture specifying process; however, possible embodiments are not limited to this example. For instance, the wearable terminal 10, the portable terminal 1, and the cloud server 5 each may be provided with the processing unit explained with reference to FIG. 3, so that the functions are arbitrarily distributed among these devices.

More specifically, the wearable terminal 10 may measure the sensor values, calculate a change amount in the height, and transmit the calculated change amount in the height to the portable terminal 10, so that the portable terminal 10 specifies the posture. Alternatively, the wearable terminal 10 may measure the sensor values and transmit the measured sensor values to the portable terminal 10, so that the portable terminal 10 calculates a change amount in the height and transmits the calculated change amount in the height to the cloud server 5, and the cloud server 5 specifies the posture. As explained herein, the processes may be distributed or integrated together in an arbitrarily manner.

### [A system]

Further, the constituent elements of the devices and apparatuses illustrated in the drawings do not necessarily have to be physically configured as indicated in the drawings. In other words, the constituent elements may be distributed or integrated together in arbitrary units. Furthermore, all or an arbitrary part of the processing functions executed by any of the devices and the apparatuses may be realized by a Central Processing Unit (CPU) and a computer program (hereinafter, "program") executed by the CPU or may be realized as hardware using wired logic.

With regard to the processes explained in the embodiments above, it is acceptable to manually perform all or a part of the processes described as being performed automatically. Conversely, by using a method that is publicly known, it is also acceptable to automatically perform all or a part of the processes described as being performed manually. Further, unless noted otherwise, it is acceptable to arbitrarily modify any of the processing procedures, the controlling procedures, specific names, and information including various types of data and parameters that are presented in the above text and the drawings.

Further, as a result of reading and executing a program, the wearable terminal 10 operates as an information processing apparatus that implements a height change amount detecting method. In other words, the wearable terminal 10 executes the program that performs the same functions as those of the acceleration obtaining unit 15, the gravity acceleration calculating unit 16, the linear acceleration calculating unit 17, the inner product unit 18, the atmospheric pressure obtaining unit 19, the change amount calculating unit 20, the multiplying unit 21, the specifying unit 22, and the notifying unit 23. As a result, the wearable terminal 10 is able to perform the same processes that implement the same functions as those of the acceleration obtaining unit 15, the gravity acceleration calculating unit 16, the linear acceleration calculating unit 17, the inner product unit 18, the atmospheric pressure obtaining unit 19, the change amount calculating unit 20, the multiplying unit 21, the specifying unit 22, and the notifying unit 23. In this situation, the program mentioned in this other embodiment does not necessarily have to be executed by the wearable terminal 10. For example, the present disclosure is similarly applicable to a situation where another computer or a server executes the program and to a situation where the program is executed by a collaboration of any of these.

### [Reference Signs List]

1 PORTABLE TERMINAL
5 CLOUD SERVER
10 WEARABLE TERMINAL
11 ACCELERATION DATA DB
12 ATMOSPHERIC PRESSURE DATA DB
13 THRESHOLD VALUE DB
14 CALCULATION RESULT DB
15 ACCELERATION OBTAINING UNIT
16 GRAVITY ACCELERATION CALCULATING UNIT
17 LINEAR ACCELERATION CALCULATING UNIT
18 INNER PRODUCT UNIT
19 ATMOSPHERIC PRESSURE OBTAINING UNIT
20 CHANGE AMOUNT CALCULATING UNIT
21 MULTIPLYING UNIT
22 SPECIFYING UNIT
23 NOTIFYING UNIT

## Claims

1. An electronic device comprising:
a first calculating unit that calculates an acceleration amount in a vertical direction by using an acceleration value;
a second calculating unit that calculates a change amount in an atmospheric pressure value by using atmospheric pressure values;
a multiplying unit that multiplies the acceleration amount in the vertical direction by the change amount in the atmospheric pressure value; and
a detecting unit that detects a change amount in the vertical direction on a basis of a multiplication result calculated by the multiplying unit.

2. The electronic device according to claim 1, wherein, when the multiplication result is equal to or larger than a predetermined value, the detecting unit sets the change amount in the vertical direction as a prescribed value and detects that no movement in the vertical direction has occurred, and when the multiplication result is smaller than the predetermined value, the detecting unit sets the multiplication result as the change amount in the vertical direction and detects that a movement in the vertical direction has occurred.

3. The electronic device according to claim 1, wherein
when the change amount in the atmospheric pressure value is a negative change amount, while the acceleration amount in the vertical direction exhibits a positive change amount, the multiplying unit calculates an absolute value of the result obtained by multiplying the acceleration amount in the vertical direction by the change amount in the atmospheric pressure value, and
the detecting unit detects the absolute value as the change amount in the vertical direction.

4. The electronic device according to claim 1, further comprising: an specifying unit that specifies a posture of a user to be one selected from among a standing position, a lying position, and a sitting position, by comparing the change amount in the vertical direction detected by the detecting unit with a threshold value.

5. The electronic device according to claim 4, wherein when the change amount in the vertical direction is smaller than a first threshold value, the specifying unit detects that the posture of the user is the lying position, and when the change amount in the vertical direction is equal to or larger than the first threshold value and smaller than a second threshold value, the specifying unit detects that the posture of the user is the sitting position.

6. The electronic device according to claim 4, wherein
when the change amount in the atmospheric pressure value is a negative change amount, while the acceleration amount in the vertical direction exhibits a positive change amount, the multiplying unit calculates an absolute value of the result obtained by multiplying the acceleration amount in the vertical direction by the change amount in the atmospheric pressure value,
the detecting unit detects the absolute value as the change amount in the vertical direction, and
when the absolute value is equal to or larger than a third threshold value, the specifying unit detects that the posture of the user is the standing position.

7. A detecting method comprising:
calculating an acceleration amount in a vertical direction by using an acceleration value;
calculating a change amount in an atmospheric pressure value by using atmospheric pressure values;
multiplying the acceleration amount in the vertical direction by the change amount in the atmospheric pressure value; and
detecting a change amount in the vertical direction on a basis of a multiplication result calculated at the multiplying.

8. A detecting computer program that causes a computer to execute a process comprising:
calculating an acceleration amount in a vertical direction by using an acceleration value;
calculating a change amount in an atmospheric pressure value by using atmospheric pressure values;
multiplying the acceleration amount in the vertical direction by the change amount in the atmospheric pressure value; and
detecting a change amount in the vertical direction on a basis of a multiplication result calculated at the multiplying.
